(19) Europäisches Patentamt
European Patent Office
Office européen des brevets

(11) EP 0 435 084 B2

(12) **NEUE EUROPÄISCHE PATENTSCHRIFT**

(45) Veröffentlichungstag und Bekanntmachung des Hinweises auf die Entscheidung über den Einspruch:
**24.09.1997 Patentblatt 1997/39**

(45) Hinweis auf die Patenterteilung:
**27.04.1994 Patentblatt 1994/17**

(21) Anmeldenummer: **90124029.1**

(22) Anmeldetag: **13.12.1990**

(51) Int. Cl.[6]: **C07C 45/50**, C07C 47/02

(54) **Verfahren zur Herstellung von Aldehyden**

Process for the production of aldehydes

Procédé de préparation d'aldéhydes

(84) Benannte Vertragsstaaten:
**AT BE CH DE ES FR GB IT LI NL SE**

(30) Priorität: **23.12.1989 DE 3942954**

(43) Veröffentlichungstag der Anmeldung:
**03.07.1991 Patentblatt 1991/27**

(73) Patentinhaber:
**HOECHST AKTIENGESELLSCHAFT**
**65926 Frankfurt am Main (DE)**

(72) Erfinder:
- **Bahrmann, Helmut, Dr. Dipl.-Chem.**
  **W-4236 Hamminkeln (DE)**
- **Fell, Bernhard, Prof. Dr. Dipl.-Chem.**
  **W-5100 Aachen (DE)**
- **Papadogianakis, Georgios, Dipl.-Chem.**
  **Heraklion / Kreta (GR)**

(56) Entgegenhaltungen:
**EP-A- 0 096 987**
**EP-A- 0 149 894**
**EP-A- 0 163 234**
**EP-A- 0 254 937**
**EP-A- 0 268 268**
**EP-A- 0 353 770**
**US-A- 4 467 116**

EP 0 435 084 B2

## Beschreibung

Die vorliegende Erfindung betrifft ein Verfahren zur Herstellung von Aldehyden durch Umsetzung olefinischer Verbindungen mit Wasserstoff und Kohlenmonoxid bei erhöhten Temperaturen und erhöhtem Druck in homogener Phase. Die Reaktion erfolgt in Gegenwart eines Rhodium sowie eine organische Phosphorverbindung enthaltenden Katalysatorsystems, wobei die organische Phosphorverbindung ein in organischen Medien lösliches Ammoniumsalz eines sulfonierten Phosphorigsäuretriesters ist.

Es ist bekannt, durch Umsetzung von Olefinen mit Kohlenmonoxid und Wasserstoff Aldehyde und Alkohole herzustellen. Allgemein wird diese, Hydroformylierung (Oxosynthese) genannte Reaktion durch Hydridometallcarbonyle, vorzugsweise solche, die sich von Metallen der Gruppe VIII A des Periodensystems ableiten, katalysiert.

Neben Kobalt, das als Katalysatormetall in großem Umfang technische Anwendung findet, gewinnt in letzter Zeit Rhodium zunehmende Bedeutung. Im Gegensatz zu Kobalt gestattet Rhodium, die Reaktion bei niedrigem Druck durchzuführen; darüberhinaus werden vorzugsweise geradkettige und nur in untergeordnetem Maß verzweigte Aldehyde gebildet. Schließlich ist auch die Hydrierung der Olefine zu gesättigten Kohlenwasserstoffen bei Anwendung von Rhodium-Katalystoren deutlich niedriger als bei Anwendung von Kobalt-Katalysatoren. Bei den in der Technik eingeführten Verfahren wird der Rhodium-Katalysator in Form modifizierter Hydridorhodiumcarbonyle eingesetzt, die zusätzliche und gegebenenfalls überschüssige Liganden enthalten. Besonders bewährt haben sich als Liganden tertiäre organische Phosphine und Phosphite.

So wird das aus $HRh(CO)(PPh_3)_3$ und überschüssigem $PPh_3$ ($Ph = C_6H_5$) bestehende Katalysatorsystem z.B. für die Hydroformylierung von alpha-Olefinen wie Ethylen, Propylen und Buten-1 bei Temperaturen zwischen 90 und 120°C eingesetzt (vgl. US 3 527 809).

In der EP 01 49 894 B1 wird die hervorragende Eignung des Rh/Triphenylphosphin-Katalysators für die Hydroformylierung von alpha-Olefinen bestätigt. Gleichzeitig wird aber darauf hingewiesen, daß die Umsetzung innenständiger Olefine wie Buten-2 Probleme aufwirft, weil sie nur bei Anwendung höherer Temperaturen zu nennenswerten Umsätzen führt. Unter diesen Bedingungen nimmt aber die Isomerisierung des Olefins stark zu. Buten-2 geht daher teilweise in Buten-1 über mit der Folge, daß bei der Hydroformylierung neben dem erwünschten 2-Methylbutyraldehyd in erheblichem Umfang auch n-Valeraldehyd gebildet wird. Gleichzeitig nehmen auch Aktivität und Stabilität des Katalysatorsystems ab, wie in der EP 00 96 987 B1 ebenfalls am Beispiel der Hydroformylierung von Buten-2 gezeigt wird. Daher ist das Katalysatorsystem Rhodium/Triphenylphosphin für die Hydroformylierung innenständiger Olefine im technischen Maßstab ungeeignet.

Die vorstehend beschriebenen Schwierigheiten lassen sich vermeiden, wenn man anstelle von Trialkyl- bzw. Triarylphosphinen Trialkyl- oder Triarylphosphite als Katalysatorkomponente einsetzt. Organische Phosphite haben nämlich den Vorteil, daß in ihrer Gegenwart die Hydroformylierung von Olefinen bei niedrigeren Temperaturen abläuft als bei Verwendung von organischen Phosphinen. So reagiert nach Beispiel 1 der EP 00 96 988 B1 Buten-2 mit Rhodium und cyclischen Phosphiten als Katalysator schon bei 2,86 MPa und 98,5°C mit Kohlenmonoxid und Wasserstoff zu 2-Methylbutyraldehyd.

Die Herstellung von Aldehyden durch Hydroformylierung von Olefinen in Gegenwart von Rhodiumverbindungen, die in komplexer Bindung Kohlenmonoxid und als Ligand ein Triaryl-, Trialkyl- oder Tricycloalkylphosphit enthalten, wobei im Reaktionsmedium mindestens 2 mol freier Ligand je g-Atom Rhodium anwesend sind, wird in der DE 17 93 069 B2 beschrieben. Beispiele betreffen sowohl die Umsetzung von Olefinen als auch von olefinisch ungesättigten Verbindungen. Als Phosphite gelangen Alkyl- und Arylverbindungen zur Anwendung.

Die Hydroformylierung von 3,3-Dialkoxy-1-propenen ist Gegenstand der DE 34 03 427 A1. Nach der beanspruchten Arbeitsweise erhält man z.B. aus 3,3-Diethoxy-1-propen in Gegenwart von Rh/Triphenylphosphit bei 110°C und 0,3 MPa Druck 3,3-Diethoxybutanal und 2-Methyl-3,3-diethoxypropanal im Molverhältnis 8,5 zu 1; der Umsatz beträgt 99,5 %.

Ebenfalls Rhodium zusammen mit Triphenylphosphit als Katalysator wird nach der EP 00 03 753 A1 bei der Umsetzung cyclischer Acroleinacetale mit Kohlenmonoxid und Wasserstoff zu den korrespondierenden Aldehyden verwendet.

Die Hydroformylierung alpha,beta-ungesättigter Nitrile wird in der US 43 44 896 beschrieben. Die Reaktion erfolgt in Gegenwart von Rhodium, das in komplexer Bindung Kohlenmonoxid und u.a. eine organische Phosphorverbindung enthält. Die Phosphorverbindung kann ein Phosphit wie Triphenylphosphit, Tri-4-tolylphosphit, Tri-4-chlorphenylphosphit, Triethylphosphit oder Tributylphosphit sein.

Zahlreiche Veröffentlichungen betreffen den Einsatz spezieller Phosphite als Bestandteil von Hydroformylierungskatalysatoren.

Der Anteil linearer Aldehyde als Produkte der Hydroformylierung von alpha- oder beta-Olefinen ist besonders hoch, wenn Rhodiumkomplexverbindungen als Katalysatoren eingesetzt werden, die fluorierte organische Phosphite als Liganden enthalten (US 43 30 678).

Nach der US 44 67 116 hydroformyliert man weniger reaktive Olefine in Gegenwart eines Metalls der Gruppe VIII A als Katalysator, das u.a. durch ein Triarylphosphit als Ligand modifiziert ist. Mindestens ein Arylrest des Phosphits ist

durch eine gegebenenfalls fluorierte Alkyl- oder durch eine Arylgruppe substituiert.

Mit der Hydroformylierung nicht reaktiver Olefine wie 2-Methyl-1-hexen, Limonen, Cyclohexen, Methylencyclohexan befassen sich auch van Leeuwen und Roobeek in J. Organomet. Chem. 258 (1983), 343 ff. Die Umsetzung solcher Olefine unter milden Bedingungen (90°, 1 MPa) gelingt in Gegenwart von Rhodiumkatalysatoren, die durch Phosphit modifiziert sind. Beispiele für die eingesetzten Phosphitliganden sind Tris(o-t-buylphenyl)phosphit und Tris(hexafluorisopropyl)phosphit; sie zeichnen sich durch spezielle sterische und elektronische Eigenschaften aus.

Auch aus EP-A-0 353 770 sind spezielle ionische Phosphite und ihre Anwendung als Liganden in homogenen, übergangsmetallkatalysierten Prozessen, insbesondere der Hydroformylierung, bekannt.

Obgleich die organischen Phosphite gegenüber den organischen Phosphinen als Bestandteil von Katalysatoren für die Oxosynthese eine Reihe Vorteile aufweisen, finden sie nur in beschränktem Umfang technische Anwendung. Ihr begrenzter Einsatz ist darauf zurückzuführen, daß die Aktivität von Rhodium/Phosphit-Katalysatoren im Laufe der Zeit nachläßt, insbesondere wenn im oberen Bereich des jeweils empfohlenen Temperaturintervalls gearbeitet wird. Gleichzeitig bilden sich in verstärktem Maße höhersiedende Verbindungen. Ursache beider Erscheinungen sind zumindest teilweise Nebenreaktionen, die das organische Phosphit in inaktive Folgeprodukte umwandeln.

In diesem Zusammenhang ist zu berücksichtigen, daß Phosphorigsäuretriester sehr hydrolyseempfindlich sind. Das während der Hydroformylierung durch Reduktionsprozesse in Spuren gebildete Wasser reicht aus, die Triester zu Di- und Monoestern und zur freien phosphorigen Säure zu verseifen. Überdies katalysieren die sauren Mono- und Diphosphite die Hydrolyse des Triesters, so daß die Reaktion autokatalytisch abläuft.

Die Hydrolysegeschwindigkeit hängt stark von der Art der Esterreste ab. Trimethylphosphit ist am instabilsten, mit zunehmender Länge der Alkylreste werden die Phosphite gegenüber hydrolytischen Einflüssen beständiger.

Die Wirksamkeit des Katalysatorsystems wird weiterhin dadurch beeinträchtigt, daß der saure Monoester den Rhodium/Phosphit-Komplex zu protonieren vermag, d.h. in eine Form umwandelt, die ebenfalls katalytisch inaktiv ist.

Die Hydrolyse der Phosphorigsäureester kann durch Zugabe organischer oder anorganischer Basen, wie z.B. tertiärer Amine (EP-A-0 149 894) zum Reaktionsgemisch verhindert oder zumindest doch verzögert werden. Nach dem Verfahren der EP 02 85 136 A1 entfernt man z.B. sekundäre organische Phosphite selektiv aus Lösungen, in denen sie zusammen mit tertiären organischen Phosphiten enthalten sind durch Zugabe eines Amins und Abtrennung des entstandenen Ammoniumphosphits. Diese Arbeitsweise läßt sich bei der Hydroformylierung olefinischer Verbindungen in Gegenwart von Rhodium/Phosphit-Katalysatoren nicht ohne weiteres anwenden. Sie erfordert die Zugabe eines reaktionsfremden Stoffes zum Reaktionsgemisch, der zu unerwünschten Folgereaktionen Anlaß geben kann.

Weitere Phosphitverluste könen durch Reaktion der Phosphorverbindung mit Aldehyden eintreten. Wie F. Ramirez zeigte (Synthesis, 1974, 90 ff), bilden Phosphorigsäuretriester mit Aldehyden bei niedriger Temperatur 4,4,4-Trialkoxy-1,3,4-dioxaphospholane, bei erhöhter Temperatur überwiegend 2,2,2-Trialkoxy-1,3,2-dioxaphospholane. Beide Verbindungsklassen sind katalytisch unwirksam, ihre Bildung beeinflußt das Verhältnis Rhodium zu Phosphit im Katalysatorsystem und führt dadurch zu einer Minderung der Aktivität des Hydroformylierungskatalysators.

Es bestand daher die Aufgabe, einen Prozeß zur Hydroformylierung olefinisch ungesättigter Verbindungen zu entwickeln, bei dem Rhodium in Kombination mit solchen Verbindungen des dreiwertigen Phosphors als Katalysator eingesetzt wird, die die Vorteile organischer Phosphine - geringe Neigung zur Reaktion mit den Bestandteilen des Reaktionsgemischs - mit denen organischer Phosphite - hohe Wirksamkeit bereits bei niedrigen Temperaturen - miteinander verbinden.

Diese Aufgabe wird gelöst durch ein Verfahren zur Herstellung von Aldehyden durch Umsetzung olefinisch ungesättigter Verbindungen mit Kohlenmonoxid und Wasserstoff in homogener Phase bei 20 bis 150°C und 0,1 bis 20 MPa in Gegenwart eines Rhodium und eine organische Phosphorverbindung enthaltenden Katalysatorsystems. Es ist dadurch gekennzeichnet, daß in dem Katalysatorsystem je g-Atom Rhodium mindestens 2 mol organische Phosphorverbindung vorliegen und als organische Phosphorverbindung ein in organischen Medien lösliches Ammmoniumsalz eines sulfonierten Phosphorigsäuretriesters

der allgemeinen Formel (2)

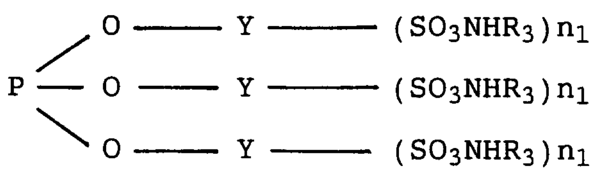

(2)

worin Y gleich oder verschieden ist und für organische Reste steht, $n_1$ gleich oder verschieden ist und eine ganze Zahl von 0 bis 4 bedeutet mit der Maßgabe, daß mindestens ein $n_1 = 1$ ist, R gleich oder verschieden ist und für aliphatische, cylcoaliphatische, aromatische, araliphatische oder heterocyclische Reste steht und vorzugsweise

ein geradkettiger oder verzweigter Alkylrest ist, wobei die drei über das Stickstoffatom mit dem Sulfonsäurerest in Verbindung stehenden Reste R zusammen 10 bis 60 und vorzugsweise 12 bis 36 Kohlenstoffatome enthalten,

der allgemeinen Formel (3)

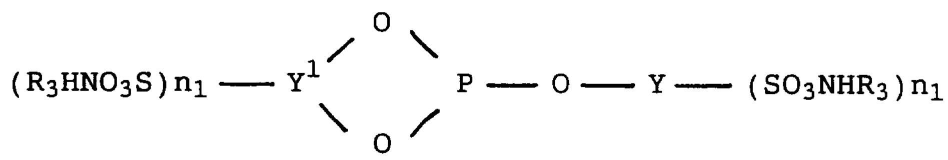

(3)

worin $Y^1$ ein organischer Rest ist, der sich von Benzol oder Naphthalin ableitet und Y, R und $n_1$ die für die allgemeine Formel (2) genannten Bedeutungen haben,

oder der allgemeinen Formel (5)

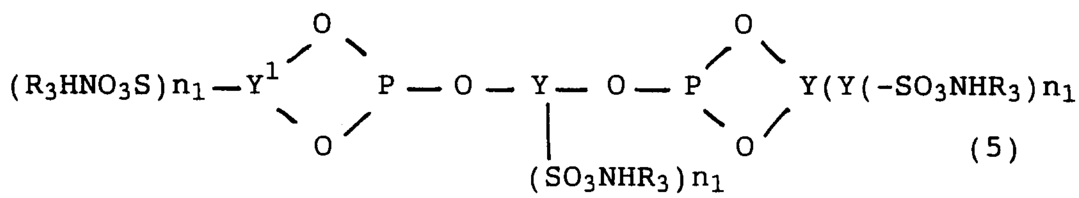

(5)

worin $Y^1$, Y R und $n_1$ die zuvor genannten Bedeutungen besitzen,

eingesetzt wird.

Überraschenderweise unterliegen die erfindungsgemäß eingesetzten organischen Phosphorverbindungen während der katalytischen Umsetzung olefinisch ungesättigter Verbindungen mit Kohlenmonoxid und Wasserstoff keinen Nebenreaktionen. Daher behält der Katalysator seine Aktivität über einen sehr langen Zeitraum unverändert bei.

In den allgemeinen Formeln (2), (3) und (5) steht Y für einen organischen Rest, z.B. für geradkettige oder verzweigte gesättigte aliphatische Reste mit 2 bis 20 Kohlenstoffatomen, für ein- oder zweikernige cycloaliphatische Reste mit 5 bis 12 Kohlenstoffatomen und für ein- oder zweikernige aromatische Reste. Die aromatischen Reste leiten sich vorzugsweise vom Benzol, vom Biphenyl, vom Naphthalin und vom Binaphthyl ab. Als Alkylarylrest hat sich vor allem der leicht zugängliche Benzylrest bewährt. Arylalkylreste gehen bevorzugt auf Toluol, Ethylbenzol und die isomeren Xylole zurück. Unter den Heterocyclen sind Reste Stickstoff enthaltender, gesättigter oder ungesättigter Fünf- oder Sechsringe von Bedeutung, insbesondere Pyridin.

Das als organische Phosphorverbindung eingesetzte, in organischen Medien lösliche Ammoniumsalz eines sulfonierten Phosphorigsäuretriesters der allgemeinen Formeln (2), (3) oder (5) weist Ammoniumionen der Allgemeinen Formel

$$[NH_xR^1_y]^+$$

auf, in der x = 1, y = 3 ist und $R^1$ für gleiche oder verschiedene aliphatische, cycloaliphatische, aromatische, araliphatische oder heterocyclische Reste steht. $R^1$ ist vorzugsweise ein geradkettiger oder verzweigter Alkylrest und die Summe aller Kohlenstoffatome in den 3 Resten $R^1$ beträgt 4 bis 60. Vorzugsweise ist die Summe der Kohlenstoffatome in den Resten $R^1$ 18 bis 42 und insbesondere 21 bis 39. Besonders geeignet sind Verbindungen, in denen $R^1$ den n-Octyl-, i-Octyl-, i-Nonyl-, i-Decyl- oder i-Tridecylrest bedeutet.

Eine Gruppe wichtiger Ammoniumsalze sulfonierter Phosphorigsäuretriester, die erfindungsgemäß eingesetzt werden, entspricht der allgemeinen Formel (2)

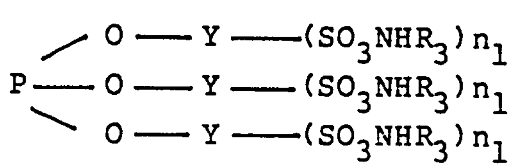

(2)

in der Y gleich oder verschieden ist und für organische Reste steht $n_1$ ist eine ganze Zahl von 0 bis 4, ebenfalls gleich oder verschieden mit der Maßgabe, daß mindestens ein $n_1 = 1$ ist. Auch R ist gleich oder verschieden und steht für aliphatische, cycloaliphatische, aromatische, araliphatische oder heterocyclische Reste. Vorzugsweise ist R ein geradkettiger oder verzweigter Alkylrest wobei die drei über das Stickstoffatom mit dem Sulfonsäurerest in Verbindung stehenden Reste R zusammen 10 bis 60 und vorzugsweise 12 bis 36 Kohlenstoffatome enthalten.

Zu den der allgemeinen Formel (2) entsprechenden Verbindungen gehören Ammoniumsulfonate von Trialkylphosphiten wie Trimethylphosphit, Triethylphosphit, Butyldiethylphosphit, Tri-n-propylphosphit, Tri-n-butylphosphit, Tri-2-ethylhexylphosphit, Tri-n-octylphosphit, Tri-n-dodecylphosphit, von Dialkylarylphosphiten wie Dimethylphenylphosphit, Diethylphenylphosphit, von Alkyldiarylphosphiten wie Methyldiphenylphosphit, Ethyldiphenylphosphit und von Triarylphosphiten wie Triphenylphosphit und Trinaphtylphosphit. Das bevorzugte Phosphit dieser Gruppe ist das Triphenylphosphittrisulfonsäure-triisooctylammoniumsalz.

Eine weitere Gruppe wichtiger Ammoniumsalze sulfonierter Phosphorigsäuretriester folgt der allgemeinen Formel (3)

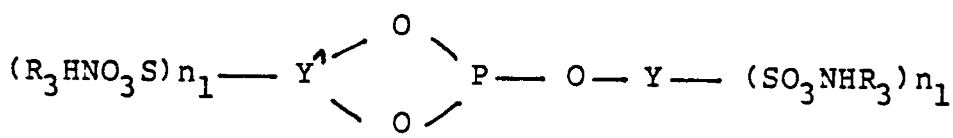

(3)

worin $Y^1$ ein organisches Rest ist, der sich von Benzol oder Naphthalin ableitet und Y die unter Formel (2) genannte Bedeutung hat und vorzugsweise für Reste steht, die sich von Benzol, Biphenyl, Naphthalin oder Binaphtyl ableiten. $n_1$ steht für die unter Formel (2) genannten ganzen Zahlen. Auch die Bedeutung von R ist unter Formel (2) wiedergegeben.

Bevorzugte Ammoniumsulfonate von Phosphiten gemäß Formel (3) sind:

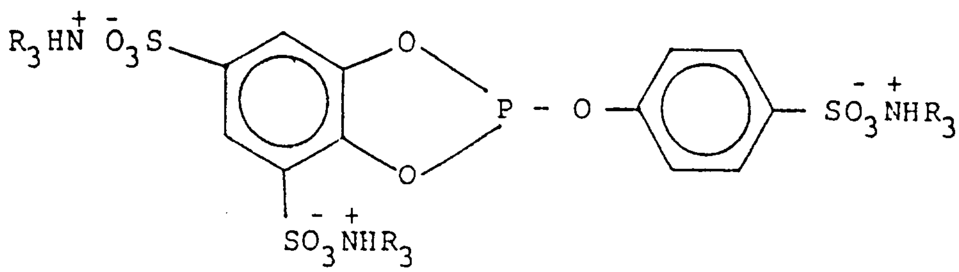

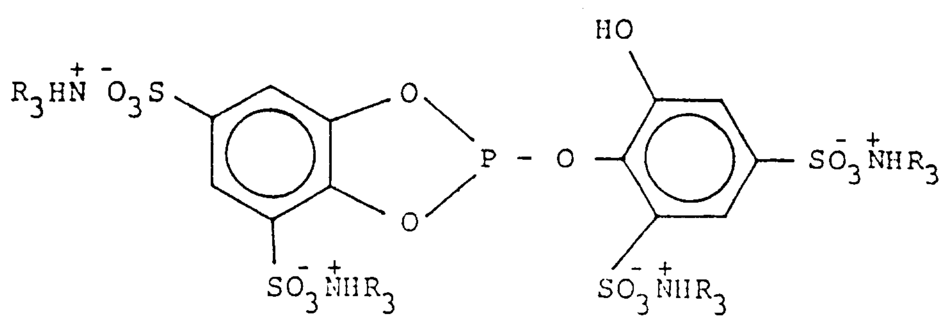

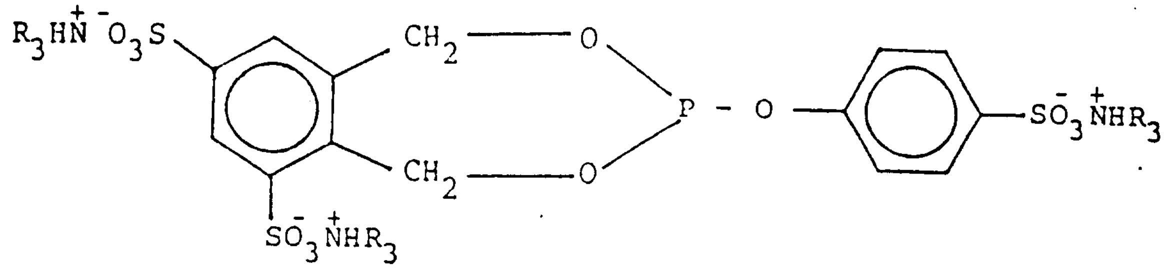

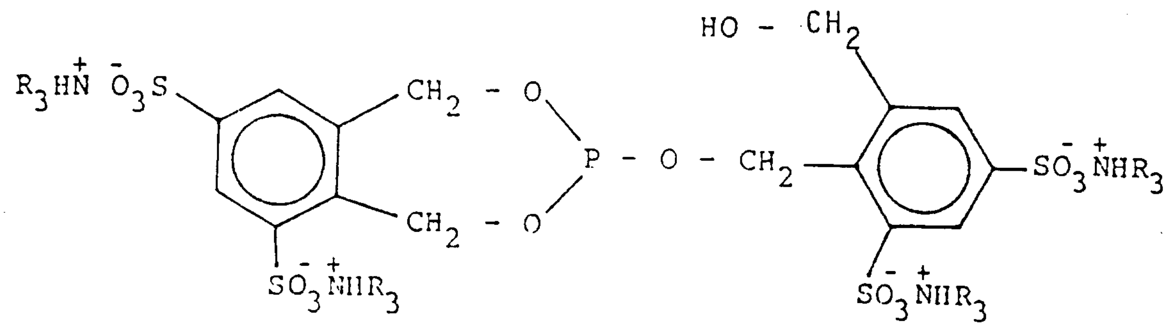

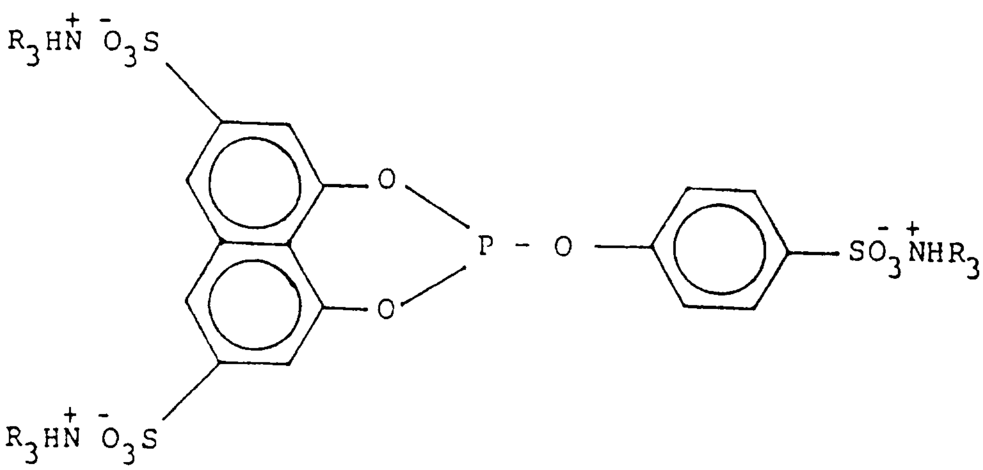

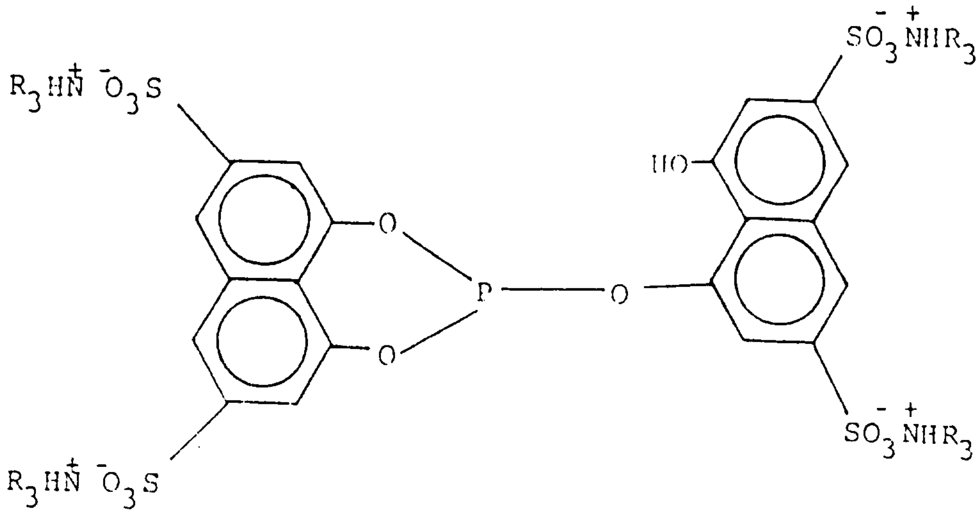

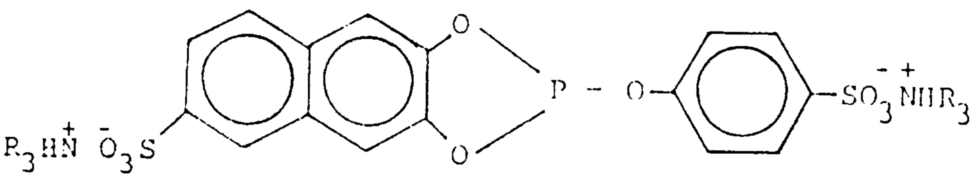

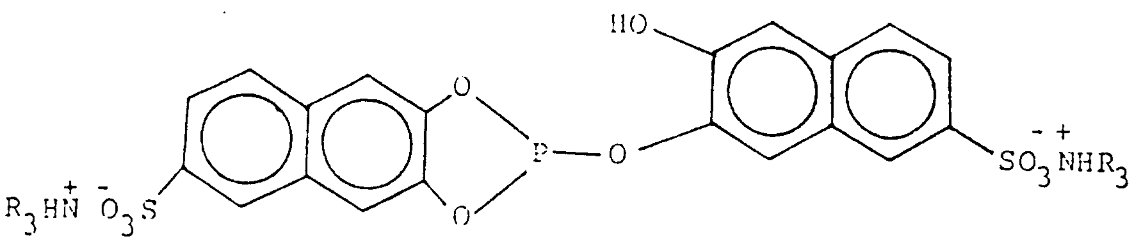

Schließlich sind auch Ammoniumsalze sulfonierter Phosphorigsäuretriester der allgemeinen Formel (6) von großer Bedeutung.

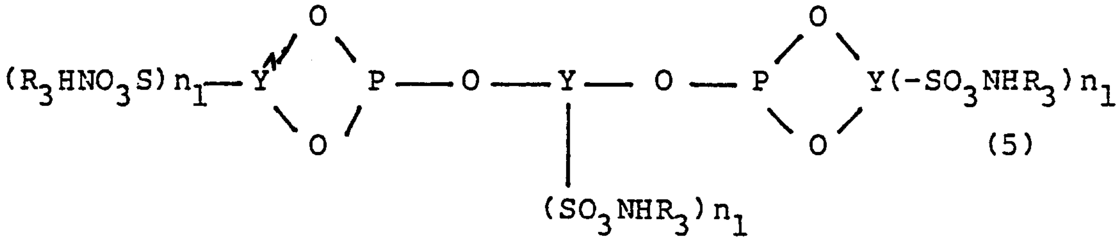

(5)

Von den in der vorstehenden allgemeinen Formel enthaltenen Symbolen steht $Y^1$ für die Formel (3) genannten Bedeutungen und Y für die unter Formel (2) wiedergegebenen organischen Reste, insbesondere für solche, die sich

vom Benzol, vom Biphenyl und vom Naphthalin sowie von Alkanen mit 2 bis 6 C-Atomen ableiten. $n_1$ und R haben die unter Formel (2) aufgeführten Bedeutungen.

Bevorzugte Ammoniumsulfonate von Phosphiten entsprechend Formel (5) sind:

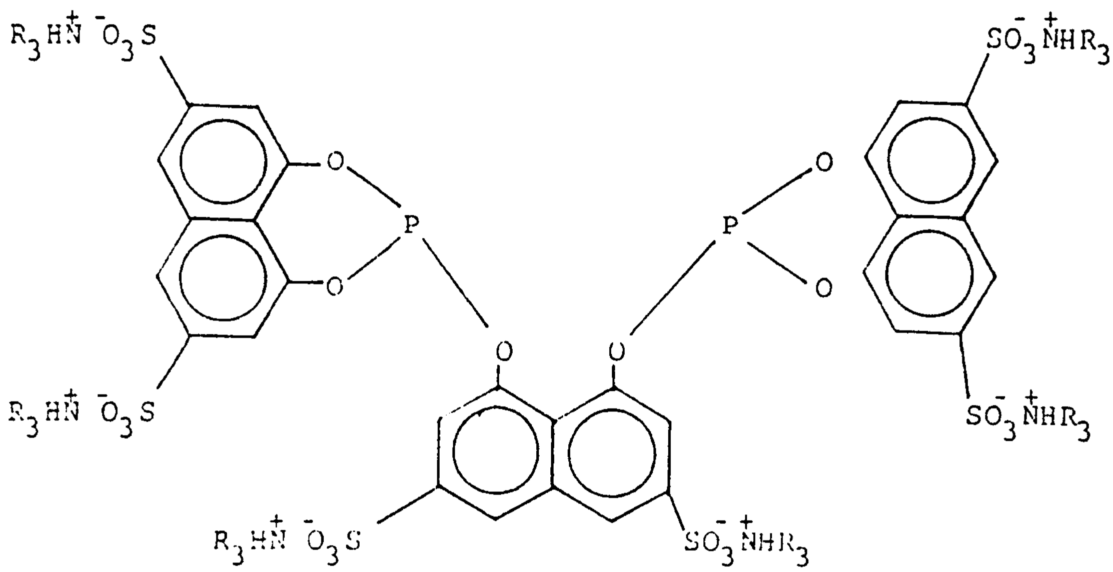

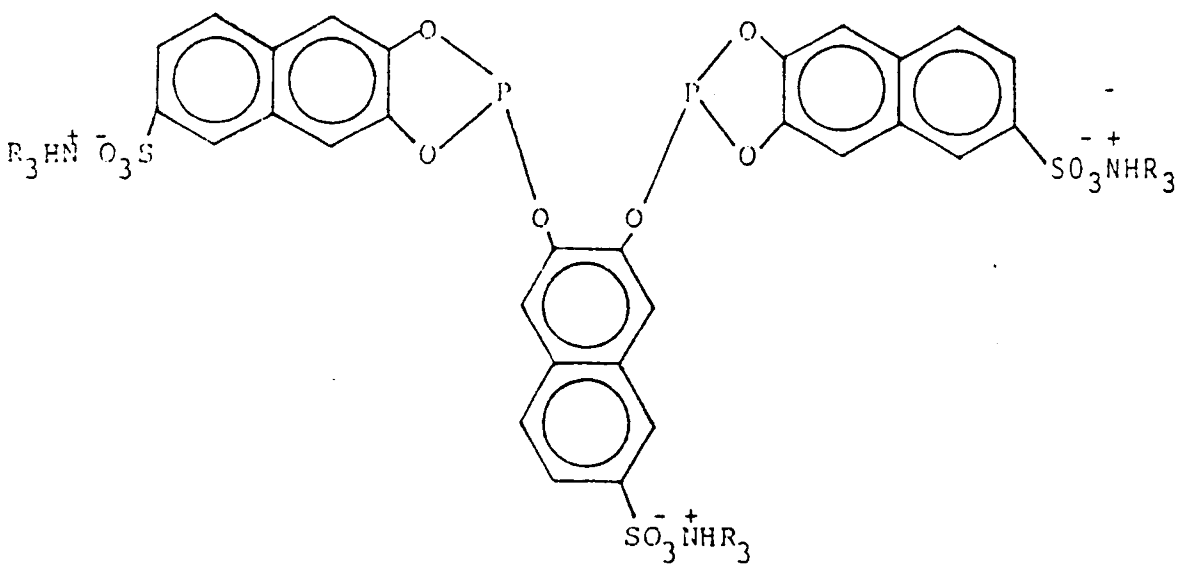

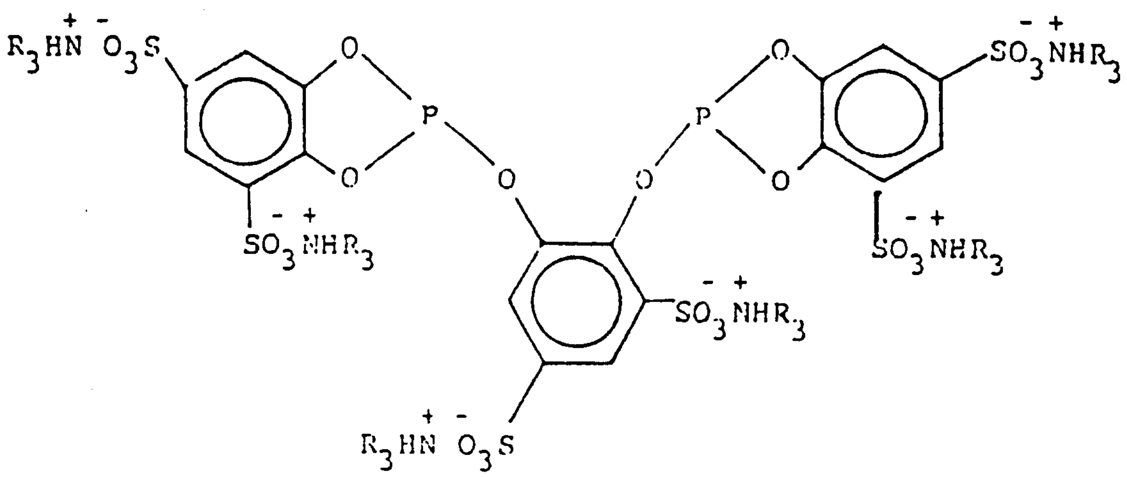

Die erfindungsgemäß eingesetzten Ammoniumsalze sulfonierter Phosphorigsäuretriester kann man durch Umesterung (Alkoholyse) von Phosphorigsäuretriestern mit dem Ammoniumsalz einer Hydroxysulfonsäure erhalten. Hierzu wird das in einem organischen Lösungsmittel gelöste Ammoniumsalz bei 20 bis 200°C, vorzugsweise 80 bis 160°C mit dem Phosphorigsäuretriester umgesetzt. Die Reaktanten werden üblicherweise in äquivalenten Mengen verwendet, wenngleich es auch möglich ist, einen der beiden Reaktionspartner im Überschuß einzusetzen. Die Reaktion wird durch Katalysatoren wie Amine, Natrium, Natriumalkoholate, Aluminiumtrichlorid, Titansäureester oder Phosphorigsäuredialkylester beschleunigt. Als Phosphorigsäuretriester haben sich Verbindungen bewährt, die sich von aliphatischen oder aromatischen Hydroxylverbindungen ableiten, vorzugsweise solchen, die 1 bis 12 Kohlenstoffatome enthalten. Beispiele für solche Phosphite sind Trimethylphosphit, Triethylphosphit, n-Butyldiethylphosphit, Tri-n-propylphosphit, Tri-n-butylphosphit, Tri-2-ethylhexylphosphit, Tri-n-octylphosphit, Tri-n-dodecyclphosphit, Dimethylphenylphosphit, Diethylphenylphosphit, Triphenylphosphit. Bevorzugtes organisches Phosphit ist das Triphenylphosphit.

Die zweite Komponente des Katalysatorsystems, das Rhodium, kann entweder als Metall, vorzugsweise auf einem Träger wie Aktivkohle, Calciumcarbonat, Tonerde oder ähnlichen Substraten oder als Rhodiumverbindung eingesetzt werden. Geeignet sind Rhodiumverbindungen, die sich unter den Reaktionsbedingungen in organischen Medien lösen. Beispiele für anorganische oder organische Rhodiumverbindungen, bei denen das Rhodium in verschiedenen Oxidationsstufen vorliegen kann, sind die Rhodiumoxide $Rh_2O$, $Rh_2O_3$, $RhO_2$, $RhO_3$, die Salze der anorganischen Wasserstoffsäuren wie die Chloride, Bromide, Jodide, Sulfide, Selenide und Telluride $RhCl_3$, $RhBr_3$, $RhJ_3$, $Rh_2S_3$, $Rh_2Se_5$, $Rh_2Te_5$, die Salze anorganischer Sauerstoffsäuren wie Rhodiumsulfit $Rh_2(SO_3)_3$, Rhodiumsulfat $Rh_2(SO_4)_3$, Rhodiumnitrat $Rh(NO_3)_3$, Rhodiumperchlorat $Rh(OH)_2ClO_4$ und Rhodiumselenat sowie Salze aliphatischer Mono- oder Polycarbonsäuren wie Rhodiumacetat $Rh(CH_3CO_2)_3$, Rhodiumpropionat, Rhodiumoxalat $Rh_2(C_2O_4)_3$, Rhodiummalonat oder Rhodium-2-ethylhexanoat. Auch Salze von Heteropolysäuren, die Rhodium enthalten, kommen in Betracht, beispielsweise Alkali- oder Erdalkalisalze, Ammoniumsalze oder Aminsalze wie Natriumhexachlororhodat $Na_3(RhCl_6)$, Kaliumhexachlororhodat $K_3(RhCl_6)$, Bariumhexachlororhodat $Ba_3(RhCl_6)_2$, Ammoniumhexachlororhodat $(NH_4)_3[RhCl_6]$, Natriumhexabromorhodat $Na_3(RhBr_6)$, Monomethylammoniumpentachlororhodat $(NH_3CH_3)_2[RhCl_5]$ und Trimethylammoniumhexachlororhodat $[NH(CH_3)_3]_3[RhCl_6]$. Weiterhin haben sich Carbonyl- und Halogencarbonylverbindungen des Rhodiums bewährt wie Tricarbonylrhodium $Rh(CO)_3$, Tetracarbonylrhodium $[Rh(CO_4]_2$, Tetrarhodiumdodekacarbonyl $Rh_4(CO)_{12}$, $[Rh(CO)_2Cl]_2$, Dicarbonylrhodiumbromid $[Rh(CO)_2]Br$ und Dicarbonylrhodiumjodid $[Rh(CO)_2]J$.

Außer einfachen können auch komplexe Salze des Rhodiums, insbesondere des dreiwertigen Rhodiums, als Katalysatorvorstufe verwendet werden. Diese Verbindungen enthalten ein-, zwei- oder dreibindige Liganden wie β-Diketone, z.B. Acetylaceton, Alkylamine, Alkyl- oder Aryldiamine, stickstoffhaltige Heterocyclen wie Pyridin, 2,2'-Dipyridin oder 1,10-Phenanthrolin, aliphatische oder cycloaliphatische und diethylenisch ungesättigte Kohlenwasserstoffe wie Cyclopentadien und 1,5-Cyclooctadien.

Fur den Einsatz im erfindungsgemäßen Verfahren besonders geeignete Rhodiumverbindungen sind $Rh_4(CO)_{12}$, $Rh_6(CO)_{16}$, $Rh(CO)_3$, $[Rh(CO)_4]_2$, $[Rh(CO)_2Cl]_2$, Rhodium-2-ethylhexanoat, Rhodium(III)-acetylacetonat, Rhodium(I)-dicarbonylacetylacetonat, Cyclooctadienylrhodiumchlorid $[Rh(C_8H_{12})Cl]_2$, Rhodiumacetat $Rh(CH_3CO_2)_3$, $[Rh(OCOCH_3)_2.H_2O]_2$, Rhodiumnitrat $Rh(NO_3)_3$ und Rhodiumoxid $Rh_2O_3$.

Der Katalysator kann als Lösung im voraus hergestellt und darauf in die Reaktionszone eingebracht werden. Beispielsweise gibt man die gewünschte Menge Rhodium zu der organischen Lösung des sulfonierten Phosphits und läßt die Ausgangsstoffe in Gegenwart von Synthesegas unter erhöhtem Druck und bei erhöhter Temperatur miteinander reagieren. Es ist auch möglich, Rhodium als Metall oder Verbindung und das sulfonierte Phosphit in einem geeigneten

organischen Medium zu lösen oder zu suspendieren und die Katalysatorlösung in situ durch einfaches Vermischen dieser Komponenten herzustellen.

Die Konzentration des Rhodiums im Reaktionsgemisch, das im wesentlichen aus Olefin, gelöstem Synthesegas, Reaktionsprodukt, Katalysator und gegebenenfalls Lösungsmittel besteht, beträgt 5 bis 500 ppm, vorzugsweise 10 bis 150 ppm Rhodium, bezogen auf das Reaktionsgemisch. Das Ammoniumsalz des sulfonierten Phosphits wird im Verhältnis 2 : 1 bis 200 : 1 vorzugsweise 10 : 1 bis 100 : 1 mol Phosphitsalz je g-Atom Rhodium eingesetzt.

Die Hydroformylierung der olefinischen Verbindungen erfolgt bei 20 bis 150°C, insbesondere von 50 bis 120°C und 0,1 bis 20 MPa, insbesondere 1 bis 10 MPa. Der Anteil olefinischer Verbindung im Reaktionsmedium ist nicht kritisch.

Die im Einzelfall anzuwendenden Reaktionsbedingungen hängen auch von der Art der olefinischen Verbindung ab. So lassen sich reaktionsfähige Einsatzstoffe bereits bei relativ niedrigen Temperaturen und Drücken in Gegenwart geringer Katalysatormengen umsetzen, während reaktionsträgere Verbindungen entsprechend schärfere Reaktionsbedingungen erfordern. Beispiele für reaktive Olefine sind n-Hexen-1, n-Octen-1, n-Decen-1, n-Dodecen-1, n-Tetradecen-1 und Styrol. Beispiele für reaktionsträge Olefine sind n-Octen-4, Tripropylen, Tetrapropylen, Dicyclopentadien und Limonen.

Die olefinische Verbindung kann als solche oder in Lösung der Hydroformylierung zugeführt werden. Geeignete Lösungsmittel sind Ketone wie Acetophenon, Aceton, Methylethylketon; niedere aliphatische Nitrile wie Acetonitril, Propionitril oder Benzonitril; Dimethylformamid; lineare oder verzweigte aliphatische gesättigte Monohydroxyverbindungen wie Methanol, Ethanol, Propanol und Isopropanol; aromatische Kohlenwasserstoffe wie Benzol oder Toluol und gesättigte cycloaliphatische Kohlenwasserstoffe wie Cyclopentan oder Cyclohexan.

Es hat sich gezeigt, daß der pH-Wert des Reaktionsgemisches während der Umsetzung absinken kann, vor allem, wenn es Wasserspuren enthält oder wenn Halogen enthaltende Rhodium-verbindungen eingesetzt werden. Vorteilhafterweise soll der pH-Wert des Reaktionsgemisches nicht weniger als 2 betragen. Allgemein wird der pH-Wert auf 2 bis 13, vorzugsweise von 4 bis 8 eingestellt. Den für die jeweilige Umsetzung zweckmäßigen pH-Wert erreicht man durch Zugabe einer bestimmten Menge einer in einem organischen Medium löslichen basischen Verbindung, beispielsweise durch Zugabe von Aminen.

Die Zusammensetzung des Synthesegases kann in weiten Grenzen variiert werden. Im allgemeinen setzt man ein Gemisch ein, in dem das Volumenverhältnis von Kohlenmonoxid zu Wasserstoff 5 : 1 bis 1 : 5 beträgt. Zweckmäßig ist dieses Verhältnis 1 : 1 oder weicht von diesem Wert nur wenig ab.

Das Verfahren kann sowohl absatzweise als auch kontinuierlich durchgeführt werden. Es wird mit Erfolg zur Hydroformylierung der verschiedensten olefinisch ungesättigten Verbindungen angewandt.

Dementsprechend können aliphatische, cycloaliphatische oder araliphatische Verbindungen mit 2 bis 20 Kohlenstoffatomen, die eine oder mehrere olefinische Doppelbindungen besitzen und gegebenenfalls auch noch funktionelle Gruppen enthalten, umgesetzt werden. Beispiele für aliphatische Verbindungen sind geradkettige oder verzweigte alpha-Olefine mit 2 bis 20 Kohlenstoffatomen wie Ethylen, Propylen, Buten-1, iso-Butylen, Penten-1, 2-Methylbuten-1, Hexen-1, Hepten-1, Octen-1, 2,4,4-Trimethylpenten-1, Nonen-1, 2-Propylhexen-1, Decen-1, Undecen-1, Dodecen-1, n-Tetradecen-1, Octadecen-1, Eicosen-1, 3-Methylbuten-1, 3-Methylpenten-1, 3-Ethyl-4-methylpenten-1, 3-Ethylhexen-1, 4,4-Dimethylnonen-1 und 6-Propyldecen-1.

Geeignet sind auch acyclische Terpene, verzweigte Olefine wie Diisobutylen, Tripropylen, Tetrapropylen, Dimersol und aliphatische Diene wie 1,3-Butadien, 1,5-Hexadien und 1,9-Decadien.

Beispiele für araliphatische Olefine sind Styrol, alpha-Methylstyrol, 1,1-Diphenylethylen, Divinylbenzol und m-Hexylstyrol.

Als cycloaliphatische Einsatzstoffe kommen z.B. Cyclooctadien, Dicyclopentadien und cyclische Terpene wie Limonen, Pinen, Camphoren und Bisabolen in Betracht.

Beispiele für olefinische Verbindungen mit funktionellen Gruppen sind Allylverbindungen, insbesondere die Alkohole und Ester wie Allylalkohol, Allylcyclohexan, Allylbenzol, 1-Allyl-4-vinylbenzol, Allylethylether, Allyl-t-butylether, Allylphenylether und Allylacetat; Vinylverbindungen, insbesondere Ester und Ether wie Vinylmethylether, Vinylethylether, β-Vinylnaphthalin, o-Vinyl-p-xylol und Vinylacetat; Acrylsäurederivate, insbesondere die Ester wie Methylacrylat, Ethylacrylat, n-Propyloct-7-enoate; Methacrylsäure-derivate, insbesondere die Ester wie Methylmethacrylat; Hex-1-en-4-ol und Oct-1-en-4-ol; Acrolein sowie Acroleinderivate wie Acroleindimethyl- und -diethylacetale; Cyanoverbindungen, insbesondere Acrylnitril und ungesättigte Ketone wie Vinylethylketon.

Mit besonderem Erfolg werden nach dem neuen Verfahren innenständige Olefine mit 4 bis 20 Kohlenstoffatomen der allgemeinen Formel R'R"C=CR'"R"", wobei R' und R'" unabhängig voneinander ein H-Atom oder ein organischer Rest sein können und zusammen auch einen cycloaliphatischen, aromatischen oder heterocyclischen Ring bilden können, R" und R"" können unabhängig voneinander ein organischer Rest sein oder zusammen einen aliphatischen, cycloaliphatischen, aromatischen oder heterocyclischen Ring bilden, hydroformyliert.

Bevorzugt werden innenständige Olefine, die halogen- und schwefelfrei sind. Beispiele für innenständige Olefine sind cis- und trans-Buten-2, 2-Methylpropen, 2-Methylbuten-2, 2,3-Dimethylbuten-2, 1,2-Diphenylethylen, Hexen-2, Hexen-3, cis- und trans-Hepten-2, Octen-2, Octen-3, Octen-4, 3-Methylhepten-2, 3-Methylhepten-3, 3-Methylhepten-5, 3,4-Dimethylhexen-2, Decen-2, Tetradecen-2, 4-Amyldecen-2, 4-Methyltridecen-2, Octadecen-2, 6,6-Dipropyldecen-3,

Prop-1-enyl-benzol, 3-Benzylhepten-3, Cyclobuten, Cyclopenten, Cyclohexen, Cyclohepten, Cycloocten, 1-Methylcyclohexen, Maleinsäurediethylester, Fumarsäurediethylester, Crotonaldehyd, Crotonaldehyddimethylacetal, Zimtsäureethylester und cis- und trans-Prop-1-enyl-t-butylether.

Die nachstehenden Beispiele erläutern die Erfindung, ohne sie auf die beschriebenen Ausführungsformen zu begrenzen. In den nachstehenden Beispielen werden folgende Abkürzungen verwendet:

| | |
|---|---|
| TPP | Triphenylphosphin |
| TPPp | Triphenylphosphit |
| TPPpS | Triphenylphosphitsulfonsäure |
| TPPpTS | Triphenylphosphit-trisulfonsäure |
| TPPpDS | Triphenylphosphit-disulfonsäure |
| TPPpMS | Triphenylphosphit-monosulfonsäure |
| TIOA | Triisooctylamin |

Unter TPPpS-TIOA wird in den folgenden Beispielen eine Mischung aus 21 Gew.-% TPPpMS-TIOA, 46 Gew.-% TPPpDS-TIOA und 33 Gew.-% TPPpTS-TIOA verstanden.

Versuchsdurchführung

Alle Versuche werden in einem mit Rührvorrichtung und Einlaßstutzen versehenen Edelstahlautoklav durchgeführt. Zur Entfernung des Sauerstoffs wird der Autoklav evakuiert, mit Argon gefüllt und auf die jeweilige Reaktionstemperatur aufgeheizt. Sodann preßt man mehrfach Synthesegas auf und entspannt wieder. Anschließend gibt man in den Autoklav die vorbereitete Katalysatorlösung, n-Tetradecen-1 und ein Drittel der berechneten Menge Acetophenon, das als Lösungsmittel verwendet wird.

Die Katalysatorlösung stellt man in einem evakuierten, ausgeheizten und mit Argon gefüllten Schlenkrohr her. In dieses Gefäß wiegt man die berechnete Menge Phosphorverbindung, gelöst in zwei Dritteln der insgesamt eingesetzten Acetophenonmenge und darauf die berechnete Menge Rhodium, als Rh-2-ethylhexanoat, ein.

Der Inhalt des Autoklav wird bei Reaktionstemperatur durch Aufpressen von Synthesegas ($CO/H_2$ = 1 : 1) auf den gewünschten Reaktionsdruck gebracht und darauf gerührt.

Der Autoklav ist mit einem Drucksensor ausgerüstet, der es gestattet, die Druckabnahme während der Reaktion kontinuierlich zu verfolgen und mittels eines Schreibers aufzuzeichnen.

Nach beendeter Reaktion (3 Stunden) wird auf etwa 25°C abgekühlt, das überschüssige Gas entspannt, der Reaktorinhalt ausgetragen und gaschromatographisch analysiert.

Die Reaktionsbedingungen und die Ergebnisse sind in den Tabellen zusammengestellt.

In allen Beispielen sind die Rhodiumkonzentrationen auf das Reaktionsgemisch bezogen.

Beispiel 1

Hydroformylierung von n-Tetradecen-1 in Gegenwart von Rhodium-2-ethylhexanoat/TPPpS-TIOA als Katalysator.

Reaktionsbedingungen:

125°C, 0,6 MPa, 3 h Reaktionszeit, $CO/H_2$ = 1 : 1,
20 g (= 0,098 mol) n-Tetradecen (94,1 % n-Tetradecen-1, 2,5 % andere n-Tetradecenisomere)
190,47 mg 1,05 %ige Rhodium-2-ethylhexanoat-Lösung (Lösungsmittel Xylol), d.h. 2 mg (= 0,0194 mmol) Rhodium; Rhodiumkonzentration: 50 ppm
Der pH-Wert liegt bei 4,2 - 4,7

in

1/1: 0,2389g (=0,1943 mmol) TPPpS-TIOA in 17,66g Acetophenon
1/2: 0,4778g (=0,3886 mmol) TPPpS-TIOA in 17,24g Acetophenon
1/3: 0,9556g (=0,7772 mmol) TPPpS-TIOA in 16,45g Acetophenon
1/4: 1,8985g (=1,544 mmol) TPPpS-TIOA in 15,02g Acetophenon
1/5: 2,3891g (=1,943 mmol) TPPpS-TIOA in 14,04g Acetophenon

Tabelle 1

| Nr. des Versuches | P/Rh-Verhältnis (in mol) | Umsatz (%) | Aldehyd-Ausbeute (%) | Selektivität (%) | | | | | | n/i-Verhältnis |
|---|---|---|---|---|---|---|---|---|---|---|
| | | | | al-1 | al-2 | al-3 | al-4 | al-5 | al-6/7 | |
| 1/1 | 10 | 34 | 33,8 | 79,52 | 19,7 | 0,7 | - | - | - | 80/20 |
| 1/2 | 20 | 34 | 33,9 | 82,2 | 17,5 | 0,2 | - | - | - | 82/18 |
| 1/3 | 40 | 30 | 29,8 | 84,8 | 15,2 | - | - | - | - | 85/15 |
| 1/4 | 80 | 9 | 8,9 | 85,9 | 14,0 | - | - | - | - | 86/14 |
| 1/5 | 100 | 7 | 6,9 | 88,1 | 11,8 | - | - | - | - | 88/12 |

Beispiel 2 (Vergleich)

Hydroformylierung von n-Tetradecen-1 in Gegenwart von Rhodium-2-ethylhexanoat/TPP als Katalysator.

Um einen direkten Vergleich zwischen TPPpS-TIOA und TPP als Cokatalysatoren zu erhalten, wird unter den Bedingungen des Beispiels 1 gearbeitet.

Reaktionsbedingungen: 125°C, 0,6 MPa, 3 h Reaktionszeit, $CO/H_2$ = 1 : 1

20 g (= 0,098 mol) n-Tetradecen (94,1 % n-Tetradecen-1, 2,5 % andere n-Tetradecenisomere)
190,47 mg 1,05 %ige Rhodium-2-ethylhexanoat-Lösung (Lösungsmittel Xylol), d.h. 2 mg (= 0,01943 mmol) Rhodium, Rhodiumkonzentration: 50 ppm

in

2/1 : 0,0509 g (= 0,1943 mmol) TPP in 17,9 g Acetophenon
2/2 : 0,1019 g (= 0,3886 mmol) TPP in 17,6 g Acetophenon
2/3 : 0,2038 g (= 0,7772 mmol) TPP in 17,8 g Acetophenon
2/4 : 0,4077 g (= 1,5544 mmol) TPP in 17,2 g Acetophenon
2/5 : 0,5096 g (= 1,943 mmol) TPP in 17,4 g Acetophenon

Tabelle 2

| Nr. des Versuches | P/Rh-Verhältnis (in mol) | Umsatz (%) | Aldehyd-Ausbeute (%) | Selektivität (%) | | | | | | n/i-Verhältnis |
|---|---|---|---|---|---|---|---|---|---|---|
| | | | | al-1 | al-2 | al-3 | al-4 | al-5 | al-6/7 | |
| 2/1 | 10 | 41 | 40,9 | 66,5 | 29,2 | 2,9 | 0,93 | 0,28 | 0,14 | 67/33 |
| 2/2 | 20 | 43 | 42,9 | 71,0 | 26,7 | 1,7 | 0,48 | 0,11 | | 71/29 |
| 2/3 | 40 | 49 | 48,9 | 73,1 | 24,8 | 1,4 | 0,4 | 0,1 | | 73/27 |
| 2/4 | 80 | 47 | 46,8 | 76,5 | 22,4 | 0,8 | 0,1 | 0,09 | | 77/23 |
| 2/5 | 100 | 44 | 43,9 | 77,9 | 21,5 | 0,4 | 0,1 | | | 78/22 |

Beispiel 3 (Vergleich)

Hydroformylierung von n-Tetradecen-1 in Gegenwart von Rhodium-2-ethylhexanoat/TPPp als Katalysator.

n-Tetradecen-1 wird mit Rh-2-ethylhexanoat und TPPp als Cokatalysator unter den Bedingungen des Beispiels 1 hydroformyliert, um die Wirkung von TPPpS-TIOA und TPPp als Cokatalysatoren vergleichen zu können.

Reaktionsbedingungen:

125°C, 0,6 MPa, 3 h Reaktionszeit, $CO/H_2$ = 1 : 1,
20 g (= 0,098 mol) n-Tetradecen (94,1 % n-Tetradecen-1, 2,5 % andere n-Tetradecenisomere)
190,47 mg 1,05 %ige Rhodium-2-ethylhexanoat-Lösung (Lösungsmittel Xylol), d.h. 2 mg (= 0,01943 mmol) Rhodium; Rhodiumkonzentration: 50 ppm

in

3/1 : 0,060 g (= 0,1943 mmol) TPPp in 17,9 g Toluol
3/2 : 0,1205 g (= 0,3886 mmol) TPPp in 18,0 g Toluol
3/3 : 0,2411 g (= 0,7772 mmol) TPPp in 17,9 g Toluol
3/4 : 0,4823 g (= 1,5544 mmol) TPPp in 17,4 g Toluol
3/5 : 0,6029 g (= 1,943 mmol) TPPp in 17,5 g Toluol

Tabelle 3

| Nr. des Versuches | P/Rh-Verhältnis (in mol) | Umsatz (%) | Aldehyd-Ausbeute (%) | Selektivität (%) | | | | | | n/i-Verhältnis |
|---|---|---|---|---|---|---|---|---|---|---|
| | | | | al-1 | al-2 | al-3 | al-4 | al-5 | al-6/7 | |
| 3/1 | 10 | 23 | 22,9 | 66,42 | 27,6 | 3,7 | 1,5 | 0,5 | 0,27 | 66/34 |
| 3/2 | 20 | 35 | 34,9 | 78,8 | 20,4 | 0,7 | 0,1 | | - | 79/21 |
| 3/3 | 40 | 47 | 46,8 | 79,7 | 19,5 | 0,6 | 0,1 | | - | 80/20 |
| 3/4 | 80 | 46 | 45,8 | 80,1 | 19,3 | 0,4 | 0,1 | | - | 80/20 |
| 3/5 | 100 | 32 | 31,9 | 82,2 | 17,6 | 0,1 | 0,1 | | - | 82/18 |

Beispiel 4

Hydroformylierung von n-Tetradecen-1 in Gegenwart von Rhodium-2-ethylhexanoat/TPPpS-TIOA als Katalysator.

Die Hydroformylierung von n-Tetradecen-1 wird unter den Bedingungen des Beispiels 1, jedoch bei pH 6, durchgeführt. Der pH-wert wird durch Zugabe von Triisooctylamin eingestellt.

Reaktionsbedingungen:

125°C, 0,6 MPa, 3 h Reaktionszeit, $CO/H_2$ = 1 : 1,
20 g (= 0,098 mol) n-Tetradecen (94,1 % n-Tetradecen-1, 2,5 % andere n-Tetradecenisomere)
190,47 mg 1,05 %ige Rhodium-2-ethylhexanoat-Lösung (Lösungsmittel Xylol), d.h. 2 mg (= 0,0194 mmol) Rhodium, Rhodiumkonzentration: 50 ppm

Der pH-wert wird durch Zugabe von 0,1 bis 0,27 g TIOA auf 6,0 eingestellt

in

4/1: 0,3131 g(=0,1943 mmol) TPPpS-TIOA in 17,4 g Acetophenon
4/2: 0,6262 g(=0,3886 mmol) TPPpS-TIOA in 17,2 g Acetophenon

4/3: 1,2524 g(=0,7772 mmol) TPPpS-TIOA in 16,1 g Acetophenon
4/4: 2,5049 g(=1,544 mmol) TPPpS-TIOA in 14,4 g Acetophenon
4/5: 3,1311 g(=1,943 mmol) TPPpS-TIOA in 13,8 g Acetophenon

Tabelle 4

| Nr. des Versuches | P/Rh-Verhältnis (in mol) | Umsatz (%) | Aldehyd-Ausbeute (%) | Selektivität (%) | | | | | | n/i-Verhältnis |
|---|---|---|---|---|---|---|---|---|---|---|
| | | | | al-1 | al-2 | al-3 | al-4 | al-5 | al-6/7 | |
| 4/1 | 10 | 51 | 50,8 | 73,8 | 22,1 | 2,6 | 1,0 | 0,4 | 0,2 | 74/26 |
| 4/2 | 20 | 48 | 47,9 | 77,2 | 20,5 | 1,7 | 0,4 | 0,2 | | 77/23 |
| 4/3 | 40 | 50 | 49,8 | 83,3 | 16,1 | 0,4 | 0,2 | | - | 83/17 |
| 4/4 | 80 | 22 | 21,9 | 83,4 | 16,6 | - | - | - | - | 83/17 |
| 4/5 | 100 | 17 | 16,9 | 87,4 | 12,6 | - | - | - | - | 87/13 |

Beispiel 5:

Hydroformylierung von n-Tetradecen-1 in Gegenwart von Rhodium-2-ethylhexanoat/TPPpS-TIOA als Katalysator.

Beispiel 1 wird wiederholt mit dem Unterschied, daß man die Rhodiumkonzentration von 50 auf 20 ppm erniedrigt und den Druck von 0,6 MPa auf 5,0 MPa erhöht.

Reaktionsbedingungen:

125°C, 5,0 MPa, 3 h Reaktionszeit, $CO/H_2$ = 1 : 1
20 g (= 0,098 mol) n-Tetradecen (93,9 % n-Tetradecen-1, 2,8 % andere n-Tetradecenisomere)
95,23 mg einer 1,05 %igen Rhodium-2-ethylhexanoat-Lösung (Lösungsmittel Xylol), d.h. 1 mg (= 0,00971 mmol) Rh, Rhodiumkonzentration: 20 ppm

in

5/1 : 0,4775g (=0,388 mmol) TPPpS-TIOA in 27,25 g Aceton
5/2 : 0,9551g (=0,7768mmol) TPPpS-TIOA in 27,77 g Aceton

Das verwendete Aceton in 5/1 und 5/2 wird nur über $CaCl_2$ vorgetrocknet.

Tabelle 5

| Nr. des Versuches | P/Rh-Verhältnis (in mol) | Umsatz (%) | Aldehyd-Ausbeute (%) | Selektivität (%) | | | | | | n/i-Verhältnis |
|---|---|---|---|---|---|---|---|---|---|---|
| | | | | al-1 | al-2 | al-3 | al-4 | al-5 | al-6/7 | |
| 5/1 | 40 | 71 | 70,9 | 73,9 | 25,3 | 0,6 | 0,14 | | - | 74/26 |
| 5/2 | 80 | 70 | 69,9 | 74,7 | 24,6 | 0,45 | 0,12 | | - | 75/25 |

Beispiel 6 (Vergleich):

Hydroformylierung von n-Tetradecen-1 in Gegenwart von Rhodium-2-ethylhexanoat/TPP als Katalysator.

Um die Wirksamkeit von TPPpS-TIOA und TPP als Cokatalysatoren zu vergleichen, wird wie in Beispiel 5 gearbeitet.

Reaktionsbedingungen:

125°C, 5,0 MPa, 3 h Reaktionszeit, $CO/H_2$ = 1 : 1,
20 g (= 0,098 mol) n-Tetradecen (93,9 % n-Tetradecen-1, 2,8 % andere n-Tetradecenisomere)
95,23 mg einer 1,05 %igen Rhodium-2-ethylhexanoat-Lösung (Lösungsmittel Xylol), d.h. 1 mg (= 0,00971 mmol) Rh, Rhodiumkonzentration: 20 ppm

in

6/1 : 0,1018 g (= 0,388 mmol) TPP in 27,76 g Aceton
6/2 : 0,2037 g (= 0,7768 mmol) TPP in 27,92 g Aceton

Tabelle 6

| Nr. des Versuches | P/Rh-Verhältnis (in mol) | Umsatz (%) | Aldehyd-Ausbeute (%) | Selektivität (%) | | | | | al-6/7 | n/i-Verhältnis |
|---|---|---|---|---|---|---|---|---|---|---|
| | | | | al-1 | al-2 | al-3 | al-4 | al-5 | | |
| 6/1 | 40 | 81 | 80,9 | 72,1 | 27,2 | 0,43 | 0,14 | | - | 72/28 |
| 6/2 | 80 | 83 | 82,9 | 72,4 | 27,1 | 0,31 | 0,13 | | - | 72/28 |

Beispiel 7:

Hydroformylierung von n-Tetradecen-1 in Gegenwart von Rhodium-2-ethylhexanoat/TPPpS-TIOA als Katalysator.

Beispiel 5 wird wiederholt mit dem Unterschied, daß die Reaktionstemperatur 110°C statt 125°C beträgt.

Reaktionsbedingungen:

110°C, 5,0 MPa, 3 h Reaktionszeit, $CO/H_2$ = 1 : 1,
20 g (= 0,098 mol) n-Tetradecen (93,9 % n-Tetradecen-1, 2,8 % andere n-Tetradecenisomere),
95,23 mg einer 1,05 %igen Rhodium-2-ethylhexanoat-Lösung (Lösungsmittel Xylol), d.h. 1 mg (0,00971 mmol) Rh, Rhodiumkonzentration: 20 ppm

in

7/1:0,4775 g (=0,3884 mmol) TPPpS-TIOA in 27,02 g Aceton
7/2:0,9551 g (=0,7768 mmol) TPPpS-TIOA in 26,7 g Aceton

Tabelle 7

| Nr. des Versuches | P/Rh-Verhältnis (in mol) | Umsatz (%) | Aldehyd-Ausbeute (%) | Selektivität (%) | | | | | n/i-Verhältnis |
|---|---|---|---|---|---|---|---|---|---|
| | | | | al-1 | al-2 | al-3 | al-4 | al-5/6 | |
| 7/1 | 40 | 90 | 89,9 | 67,7 | 29,4 | 2,1 | 0,49 | 0,15 | 68/32 |
| 7/2 | 80 | 88 | 87,9 | 70,8 | 28,1 | 0,8 | 0,1 | | 71/29 |

Beispiel 8 (Vergleich):

Hydroformylierung von n-Tetradecen-1 in Gegenwart von Rhodium-2-ethylhexanoat/TPP als Katalysator.

Um die Wirksamkeit von TPPpS-TIOA und TPP als Cokatalysatoren bei niedrigeren Temperaturen zu vergleichen, wird wie in Beispiel 7 gearbeitet.

Reaktionsbedingungen:

110°C, 5,0 MPa, 3 h Reaktionszeit, $CO/H_2$ = 1 : 1;
20 g (= 0,098 mol) n-Tetradecen (93,9 % n-Tetradecen-1 und 2,8 % andere n-Tetradecenisomere);
95,23 mg einer 1,05 %igen Rhodium-2-ethylhexanoat-Lösung (Lösungsmittel Xylol), d.h. 1 mg (= 0,00971 mmol) Rh, Rhodiumkonzentration: 20 ppm

in

8/1 : 0,1018 g (= 0,3084 mmol) TPP in 27,98 g Aceton
8/2 : 0,2037 g (= 0,7768 mmol) TPP in 27,75 g Aceton

Tabelle 8

| Nr. des Versuches | P/Rh-Verhältnis (in mol) | Umsatz (%) | Aldehyd-Ausbeute (%) | Selektivität (%) | | | | n/i-Verhältnis |
|---|---|---|---|---|---|---|---|---|
| | | | | al-1 | al-2 | al-3 | al-4/5 | |
| 8/1 | 40 | 69 | 68,9 | 72,6 | 27,1 | 0,12 | 0,09 | 73/27 |
| 8/2 | 80 | 63 | 62,9 | 72,6 | 27,1 | 0,1 | 0,1 | 73/27 |

Beispiel 9 (Vergleich):

Hydroformylierung von n-Tetradecen-1 in Gegenwart von Rhodium-2-ethylhexanoat/TPPp als Katalysator.

In diesem Beispiel wird die Wirksamkeit von TPPp als Cokatalysator (Beispiele 7 und 8) bei der Hydroformylierung von n-Tetradecen-1 unter den Bedingungen der Beispiele 7 und 8 untersucht.

Reaktionsbedingungen:

110°C, 5,0 MPa, 3 h Reaktionszeit, $CO/H_2$ = 1 : 1;
20 g (= 0,098 mol) n-Tetradecen (93,9 % n-Tetradecen-1 und 2,8 % andere n-Tetradecenisomere);
95,23 mg einer 1,05 %igen Rhodium-2-ehylhexanoat-Lösung (Lösungsmittel Xylol), d.h. 1 mg (= 0,00971 mmol) Rh, Rhodiumkonzentration: 20 ppm

in

9/1 : 0,1205 g (= 0,388 mmol) TPPp in 28,03 g Aceton
9/2 : 0,2410 g (= 0,7768 mmol) TPPp in 27,82 g Aceton

Tabelle 9

| Nr. des Versuches | P/Rh-Verhältnis (in mol) | Umsatz (%) | Aldehyd-Ausbeute (%) | Selektivität (%) | | | | | | n/i-Verhältnis |
|---|---|---|---|---|---|---|---|---|---|---|
| | | | | al-1 | al-2 | al-3 | al-4 | al-5 | al-6/7 | |
| 9/1 | 40 | 81 | 80,9 | 71,2 | 27,8 | 0,6 | 0,04 | 0,04 | 0,16 | 71/29 |
| 9/2 | 80 | 77 | 76,9 | 72,7 | 26,9 | 0,2 | 0,1 | | - | 73/27 |

Beispiel 10:

Hydroformylierung von n-Hexen-1 in Gegenwart von $Rh_4(CO)_{12}$/TPPpS-TIOA als Katalysator.
Beispiel 1 wird wiederholt mit dem Unterschied, daß man n-Hexen-1 einsetzt und bei 2,5 MPa arbeitet.

Reaktionsbedingungen:

125°C, 2,5 MPa, 3 h Reaktionszeit, $CO/H_2$ = 1 : 1;
16,66 g (= 0,194 mol) n-Hexen (97,07 % aus n-Hexen-1 und 0,87 % andere n-Hexenisomere);
1,81 ml einer $Rh_4(CO)_{12}$-Lösung (300 mg $Rh_4(CO)_{12}$ in 300 ml Toluol), d.h. 1 mg (= 0,00971 mmol) Rh, Rhodiumkonzentration: 20 ppm

in

10/1: 0,1194 g(=0,09717mmol)TPPpS-TIOA in 29,56g Acetophenon
10/2: 0,4779 g(=0,3887 mmol)TPPpS-TIOA in 27,80g Acetophenon
10/3: 0,9558 g(=0,7774 mmol)TPPpS-TIOA in 26,20g Acetophenon
10/4: 1,1948 g(=0,9717 mmol)TPPpS-TIOA in 25,86g Acetophenon

Tabelle 10

| Nr. des Versuches | P/Rh-Verhältnis (in mol) | Umsatz (%) | Aldehyd-Ausbeute (%) | Selektivität (%) | | | n/i-Verhältnis |
|---|---|---|---|---|---|---|---|
| | | | | n-Heptanal | 2-Methylhexanal | 2-Ethylpentanal | |
| 10/1 | 10 | 89 | 88,7 | 60,0 | 32,3 | 7,5 | 60/40 |
| 10/2 | 40 | 88 | 87,4 | 75,5 | 23,0 | 1,4 | 76/24 |
| 10/3 | 80 | 90 | 89,9 | 78,6 | 20,6 | 0,7 | 79/21 |
| 10/4 | 100 | 82 | 81,7 | 79,9 | 19,6 | 0,4 | 80/20 |

Beispiel 11 (Vergleich):

Hydroformylierung von n-Hexen-1 in Gegenwart von $Rh_4(CO)_{12}$/TPP, als Katalysator.

Um die Wirksamkeit von TPPpS-TIOA und TPP als Cokatalysatoren zu vergleichen, wird wie in Beispiel 10 gearbeitet.

Reaktionsbedingungen:

125°C, 2,5 MPa, 3 h Reaktionszeit, $CO/H_2$ = 1 : 1;
16,66 g (= 0,194 mol) n-Hexen (97,07 % n-Hexen-1 und 0,87 % andere n-Hexenisomere);
1,81 ml einer $Rh_4(CO)_{12}$-Lösung (300 mg $Rh_4(CO)_{12}$ in 300 ml Toluol), d.h. 1 mg (= 0,009717 mmol) Rh, Rhodiumkonzentration: 20 ppm

in

11/1 : 0,025 g (= 0,09717 mmol) TPP in 30,25 g Toluol
11/2 : 0,1019 g (= 0,3887 mmol) TPP in 29,90 g Toluol
11/3 : 0,2548 g (= 0,9717 mmol) TPP in 29,80 g Toluol

Tabelle 11

| Nr. des Versuches | P/Rh-Verhältnis (in mol) | Umsatz (%) | Aldehyd-Ausbeute (%) | Selektivität (%) | | | n/i-Verhältnis |
|---|---|---|---|---|---|---|---|
| | | | | n-Heptanal | 2-Methylhexanal | 2-Ethylpentanal | |
| 11/1 | 10 | 89 | 88,5 | 67,5 | 29,5 | 2,9 | 68/32 |
| 11/2 | 40 | 86 | 85,7 | 72,3 | 27,1 | 0,6 | 72/28 |
| 11/3 | 100 | 85,5 | 85,4 | 74,6 | 25,3 | 0,1 | 75/25 |

Beispiel 12 (Vergleich):

Hydroformylierung von n-Hexen-1 in Gegenwart von $Rh_4(CO)_{12}$/TPPp als Katalysator.

n-Hexen-1 wird mit $Rh_4(CO)_{12}$ und Triphenylphosphit (TPPp) als Cokatalysator unter gleichen Bedingungen wie in den Beispielen 10 und 11 hydroformyliert, um die Wirksamkeit von TPPpS-TIOA und TPP als Cokatalysator zu vergleichen.

Reaktionsbedingungen:

125°C, 2,5 MPa, 3 h Reaktionszeit, $CO/H_2$ = 1 : 1;
16,66 g (= 0,194 mol) n-Hexen (97,07 % n-Hexen-1, 0,87 % andere n-Hexenisomere);
1,81 ml einer $Rh_4(CO)_{12}$-Lösung (300 mg $Rh_4$ $(CO)_{12}$ in 300 ml Toluol), d.h. 1 mg (= 0,00971 mmol) Rh, Rhodiumkonzentration: 20 ppm

in

12/1 : 0,030 g (= 0,0971 mmol) TPPp in 30,0 g Toluol
12/2 : 0,120 g (= 0,3387 mmol) TPPp in 29,8 g Toluol
12/3 : 0,301 g (= 0,9717 mmol) TPPp in 26,5 g Toluol

Tabelle 12

| Nr. des Versuches | P/Rh-Verhältnis (in mol) | Umsatz (%) | Aldehyd-Ausbeute (%) | Selektivität (%) | | | n/i-Verhältnis |
|---|---|---|---|---|---|---|---|
| | | | | n-Heptanal | 2-Methylhexanal | 2-Ethylpentanal | |
| 12/1 | 10 | 65 | 65 | 45,5 | 41,3 | 13,2 | 45/55 |
| 12/2 | 40 | 92 | 91,7 | 67,6 | 27,9 | 4,4 | 68/32 |
| 12/3 | 100 | 87 | 87 | 74,4 | 23,7 | 1,9 | 74/26 |

## Patentansprüche

1. Verfahren zur Herstellung von Aldehyden durch Umsetzung olefinisch ungesättigter Verbindungen mit Kohlenmonoxid und Wasserstoff in homogener Phase bei 20 bis 150°C und 0,1 bis 20 MPa in Gegenwart eines Rhodium und eine organische Phosphorverbindung enthaltenden Katalysatorsystems, dadurch gekennzeichnet, daß in dem Katalysatorsystem je g-Atom Rhodium mindestens 2 mol organische Phosphorverbindung vorliegen und als organische Phosphorverbindung ein in organischen Medien lösliches Ammoniumsalz eines sulfonierten Phosphorigsäuretriesters

   der allgemeinen Formel (2)

   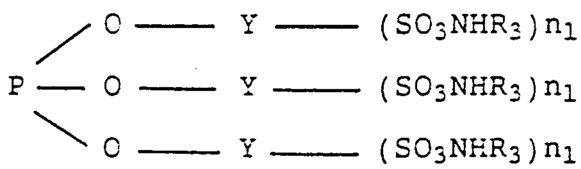

   (2)

   worin Y gleich oder verschieden ist und für organische Reste steht, $n_1$ gleich oder verschieden ist und eine ganze Zahl von 0 bis 4 bedeutet mit der Maßgabe, daß mindestens ein $n_1 = 1$ ist, R gleich oder verschieden ist und für aliphatische, cycloaliphatische, aromatische, araliphatische oder heterocyclische Reste steht und vorzugsweise ein geradkettiger oder verzweigter Alkylrest ist, wobei die drei über das Stickstoffatom mit dem Sulfonsäurerest in Verbindung stehenden Reste R zusammen 10 bis 60 und vorzugsweise 12 bis 36 Kohlenstoffatome enthalten,

   der allgemeinen Formel (3)

   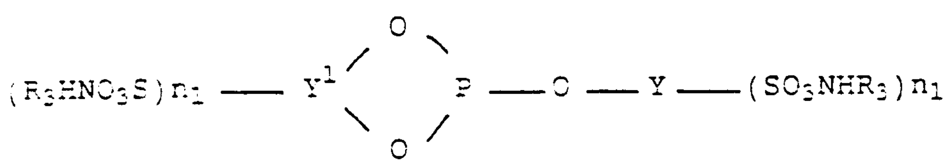

   (3)

   worin $Y^1$ ein organischer Rest ist, der sich von Benzol oder Naphthalin ableitet und Y, R und $n_1$ die für die allgemeine Formel (2) genannten Bedeutungen haben,

   oder der allgemeinen Formel (5)

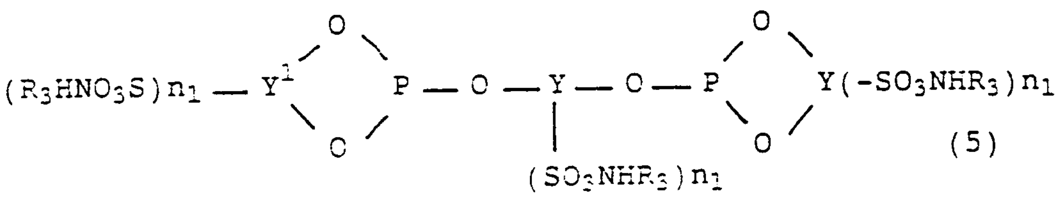

(5)

worin $Y^1$, Y, R und $n_1$ die zuvor genannten Bedeutungen besitzen,

eingesetzt wird.

2. Verfahren nach Anspruch 1, dadurch gekennzeichnet, daß Y ein geradkettiger oder verzweigter, gesättigter aliphatischer Rest mit 2 bis 20 Kohlenstoffatomen, ein ein- oder zweikerniger cycloaliphatischer Rest mit 5 bis 12 Kohlenstoffatomen, ein ein- oder zweikerniger aromatische Rest, der sich vorzugsweise vom Benzol, vom Biphenyl, vom Naphthalin oder vom Binaphthyl ableitet, ein Alkylarylrest, insbesondere ein Benzylrest, ein Arylalkylrest, der vorzugsweise auf Toluol, Ethylbenzol oder die isomeren Xylole zurückgeht oder ein Stickstoff enthaltender, gesättigter oder ungesättigter, heterocyclischer Fünf- oder Sechsring, insbesondere Pyridin, ist.

3. Verfahren nach Anspruch 1 oder 2, dadurch gekennzeichnet, daß das als organische Phosphorverbindung eingesetzte, in organischen Medien lösliche Ammoniumsalz eines sulfonierten Phosphorigsäuretriesters Ammoniumionen der allgemeinen Formel

$$[NH_xR^1_y]^+$$

aufweist, in der x = 1, y = 3 ist und $R^1$ für gleiche oder verschiedene aliphatische, cycloaliphatische, aromatische, araliphatische oder heterocyclische Reste steht.

4. Verfahren nach Anspruch 3, dadurch gekennzeichnet, daß $R^1$ ein geradkettiger oder verzweigter Alkylrest ist und die Summe aller Kohlenstoffatome in den 3 Resten $R^1$ 4 bis 60, vorzugsweise 18 bis 42, insbesondere 21 bis 39 ist.

5. Verfahren nach Anspruch 4, dadurch gekennzeichnet, daß $R^1$ den n-Octyl, i-Octyl, i-Nonyl, i-Decyl- oder i-Tridecylrest bedeutet.

6. Verfahren nach einem oder mehreren der Ansprüche 1 bis 5, dadurch gekennzeichnet, daß Rhodium in einer Menge von 5 bis 500 ppm, vorzugsweise 10 bis 150 ppm, bezogen auf das Reaktionsgemisch, verwendet wird und je g-Atom Rhodium mindestens 2 mol Phosphorigsäuretriester eingesetzt werden.

7. Verfahren nach Anspruch 6, dadurch gekennzeichnet, daß je g-Atom Rhodium 2 bis 200, vorzugsweise 10 bis 100 mol Phosphorigsäuretriester eingesetzt werden.

8. Verfahren nach einem oder mehreren der Ansprüche 1 bis 7, dadurch gekennzeichnet, daß die Umsetzung bei Temperaturen von 50 bis 120°C und Drücken von 1 bis 10 MPa erfolgt.

9. Verfahren nach einem oder mehreren der Ansprüche 1 bis 8, dadurch gekennzeichnet, daß der pH-wert des Reaktionsgemisches 2 bis 13, vorzugsweise 4 bis 8 beträgt.

10. Verfahren nach einem oder mehreren der Ansprüche 1 bis 9, dadurch gekennzeichnet, daß das Volumverhältnis von Kohlenmonoxid zu Wasserstoff 5 : 1 bis 1 : 5, vorzugsweise etwa 1 : 1 beträgt.

**Claims**

1. A process for the preparation of aldehydes by reaction of olefinically unsaturated compounds with carbon monoxide and hydrogen in the homogeneous phase at 20 to 150°C and 0.1 to 20 MPa in the presence of a catalyst system containing rhodium and an organic phosphorus compound, characterised in that the catalyst system contains at least 2 mol of organic phosphorus compound per g-atom of rhodium and uses, as the organic phosphorus compound, an ammonium salt, soluble in organic media, of a sulfonated phosphorous acid triester of the general formula (2)

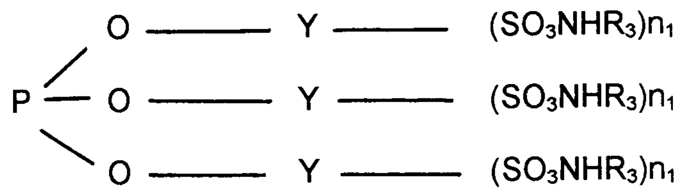

(2)

where Y is the same or different and denotes organic radicals, $n_1$ is the same or different and is an integer from 0 to 4, with the proviso that at least one $n_1$ is 1 and R is the same or different and stands for aliphatic, cycloaliphatic, aromatic, araliphatic or heterocyclic radicals and is preferably a straight-chain or branched alkyl radical, the three radicals R joined to the sulfonic acid radical via the nitrogen atom together containing 10 to 60 and preferably 12 to 36 carbon atoms of the general formula (3)

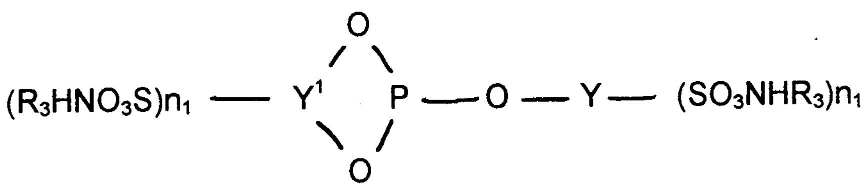

(3)

where $Y^1$ is an organic radical which is derived from benzene or naphthalene and Y, R and $n_1$ denote the same as mentioned in the general formula (2), or of the general formula (5)

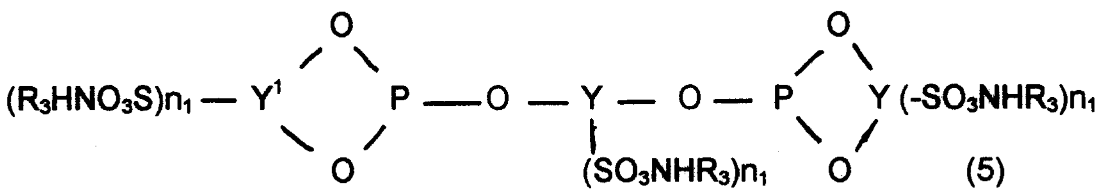

(5)

where $Y_1$, Y, R and $n_1$ denote the same as mentioned hereinabove.

2. Process according to claim 1, characterised in that Y is a straight-chain or branched, saturated aliphatic radical having 2 to 20 carbon atoms, a mono or dinuclear cycloaliphatic radical having 5 to 12 carbon atoms, a mono or dinuclear aromatic radical, which is preferably derived from benzene, biphenyl, naphthalene or binaphthyl, an alkylaryl radical, in particular a benzyl radical, an arylalkyl radical, which is preferably based on toluene, ethylbenzene or the isomeric xylenes, or a nitrogen-containing, saturated or unsaturated, heterocyclic five or six-membered ring, in particular pyridine.

3. Process according to claim 1 or 2, characterised in that the ammonium salt of a sulfonated phosphorous acid triester, which is soluble in organic media and used as the organic phosphorus compound, contains ammonium ions of the general formula

$$[NH_xR^1_y]^+$$

where x = 1 and y = 3 and $R^1$ denotes the same or different aliphatic, cycloaliphatic, aromatic, araliphatic or heterocyclic radicals.

4. Process according to claim 3, characterised in that $R^1$ is a straight-chain or branched alkyl radical and the sum of all the carbon atoms in the radicals $R^1$ is 4 to 60, preferably 18 to 42, in particular 21 to 39.

5. Process according to claim 4, characterised in that $R^1$ is the n-octyl, i-octyl, i-nonyl, i-decyl or i-tridecyl radical.

6. Process according to one or more of claims 1 to 5, characterised in that rhodium is used in an amount of 5 to 500 ppm, preferably 10 to 150 ppm, related to the reaction mixture, and at least 2 mol of phosphorous acid triester are

used per g-atom of rhodium.

7. Process according to claim 6, characterised in that 2 to 200, preferably 10 to 100 mol of phosphorous acid triester are employed per g-atom of rhodium.

8. Process according to one or more of claims 1 to 7, characterised in that the reaction is carried out at temperatures of 50 to 120°C and pressures of 1 to 10 MPa.

9. Process according to one or more of claims 1 to 8, characterised in that the pH of the reaction mixture is 2 to 13, preferably 4 to 8.

10. Process according to one or more of claims 1 to 9, characterised in that the volume ratio of carbon monoxide to hydrogen is 5 : 1 to 1 : 5, preferably about 1 : 1.

## Revendications

1. Procédé pour préparer des aldéhydes par la réaction de composés à insaturation oléfinique avec du monoxyde de carbone et de l'hydrogène en phase homogène entre 20 et 150°C et sous une pression de 0,1 à 20 MPa en présence d'un système de catalyseur contenant du rhodium et un composé organique du phosphore, caractérisé en ce que, dans le système de catalyseur, il y a pour chaque atome-g de rhodium au moins 2 mol du composé organique du phosphore et en ce qu'on utilise comme composé organique du phosphore un sel d'ammonium, soluble dans les milieux organiques, d'un triester d'acide phosphoreux sulfoné

de formule générale (2):

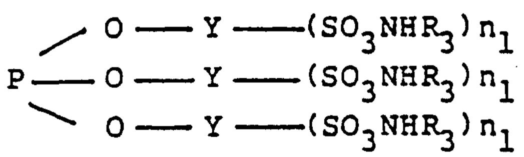

(2)

dans laquelle Y est identique ou différent et représente des restes organiques, $n_1$ est identique ou différent et représente un entier de 0 à 4, à la condition qu'au moins un $n_1 = 1$, R est identique ou différent et représente des restes aliphatiques, cycloaliphatiques, aromatiques, araliphatiques ou hétérocycliques et est de préférence un reste alkyle linéaire ou ramifié, les trois restes R reliés par l'atome d'azote au reste acide sulfonique contenant ensemble 10 à 60 et de préférence 12 à 36 atomes de carbone,
de formule générale (3):

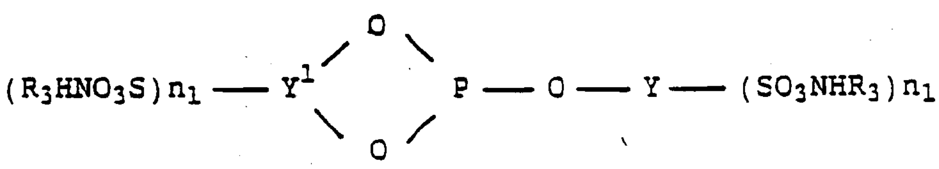

(3)

dans laquelle $Y^1$ est un reste organique qui dérive du benzène ou du naphtalène et Y, R et $n_1$ ont les significations données dans le cas de la formule générale (2)
ou de formule générale (5):

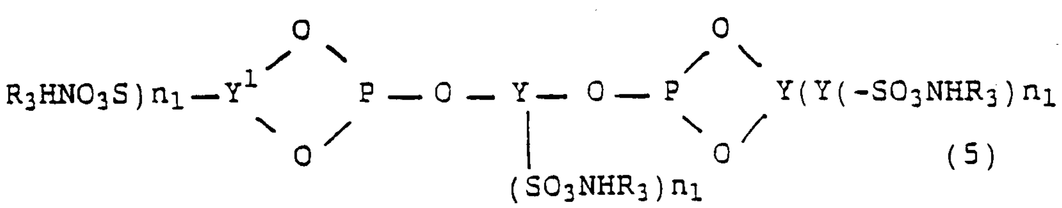

(5)

dans laquelle $Y^1$, Y, R et $n_1$ ont les significations données précédemment.

2. Procédé selon la revendication 1, caractérisé en ce que Y est un reste aliphatique saturé, linéaire ou ramifié, comportant 2 à 20 atomes de carbone, un reste cycloaliphatique monocyclique ou bicyclique comportant 5 à 12 atomes de carbone, un reste aromatique monocyclique ou bicyclique qui dérive de préférence du benzène, du biphényle, du naphtalène ou du binaphtyle, un reste alkylaryle, notamment un reste benzyle, un reste arylalkyle qui dérive de préférence du toluène, de l'éthylbenzène ou des xylènes isomères ou un reste hétérocyclique pentagonal ou hexagonal, saturé ou insaturé, contenant de l'azote, notamment de la pyridine.

3. Procédé selon la revendication 1 ou 2, caractérisé en ce que le sel d'ammonium soluble dans des milieux organiques, utilisé comme composé organique du phosphore, d'un triester d'acide phosphoreux sulfoné présente des ions ammonium de formule générale :

$$[NH_xR^1_y]^+$$

dans laquelle x = 1; y = 3; et $R^1$ représente des restes aliphatiques, cycloaliphatiques, aromatiques, araliphatiques ou hétérocycliques identiques ou différents.

4. Procédé selon la revendication 3, caractérisé en ce que $R^1$ représente un reste alkyle linéaire ou ramifié et la somme de tous les atomes de carbone dans les 3 restes $R^1$ est de 4 à 60, de préférence de 18 à 42, notamment de 31 à 39.

5. Procédé selon la revendication 4, caractérisé en ce que $R^1$ représente le reste n-octyle, i-octyle, i-nonyle, i-décyle ou i-tridécyle.

6. Procédé selon une ou plusieurs des revendications 1 à 5, caractérisé en ce qu'on utilise le rhodium en une quantité de 5 à 500 ppm, de préférence de 10 à 150 ppm, par rapport au mélange réactionnel, et en ce qu'on utilise, pour chaque atome-g de rhodium, au moins 2 mol de triester d'acide phosphoreux.

7. Procédé selon la revendication caractérisé en ce qu'on utilise pour chaque atome-g de rhodium 2 à 200, de préférence 10 à 100 mol, de triester d'acide phosphoreux.

8. Procédé selon une ou plusieurs des revendications 1 à 7, caractérisé en ce que la réaction a lieu à des températures de 50 à 120°C et sous des pressions de 1 à 10 MPa.

9. Procédé selon une ou plusieurs des revendications 1 à 8, caractérisé en ce que la valeur du pH du mélange réactionnel est de 2 à 13, de préférence de 4 à 8.

10. Procédé selon une ou plusieurs des revendications 1 à 14, caractérisé en ce que le rapport en volume du monoxyde de carbone à l'hydrogène est de 5:1 à 1:5, de préférence d'environ 1:1.